# EUROPEAN PATENT APPLICATION

(11) **EP 3 925 557 A1**
(43) Date of publication of application: **22.12.2021**
(21) Application number: 21179591.9
(22) Date of filing: 15.06.2021
(51) Int. Cl.: A61B 17/70, A61B 90/00, A61B 17/86, A61B 17/88

(54) **VERTEBRAL STABILIZATION SYSTEM AND APPARATUS AND POSITIONING TOOL FOR POSITIONING THIS STABILIZATION APPARATUS**

(30) Priority: 15.06.2020 IT 202000014185
(71) Applicant: Leader Medica S.r.l., 35129 Padova (IT); Innovazione e Progetti Sanit' S.r.l., 00198 Roma (IT)
(72) Inventor: Agrillo, Umberto, 00144 Roma (IT)
(74) Representative: Morabito, Sara

(57) **Abstract**

A vertebral stabilization apparatus (200) comprises at least one fixing screw (1) which is intended to be fixed to a vertebral body (101), a saddle-like head (3) which is intended to receive the fixing screw (1) in a tiltable manner and which defines a receiving seat (7) and a locking element (10) which is intended to be inserted in the receiving seat (7) in order to lock the at least one fixing screw (1) in a desired configuration, the locking element (10) having a virtually cylindrical body (11) which has a central cavity (12) which extends along the longitudinal axis (X) of the locking element (10), the body (11) being provided on an external surface thereof with a positioning thread (15) which is intended to be screwed to the receiving seat (7) and with at least two longitudinal engagement cavities (16) for a screwing tool (30), the central cavity (12) comprising a first cavity portion (21) which is shaped so as to define a locking seat (23) and a second cavity portion (22) which is positioned beside the first cavity portion (21) in a longitudinal direction (X) and which is delimited by a wall portion (20B) which is provided with a circumferential channel (24) which defines an engagement seat (24) for an engaging tool (40, 45) of the locking element (10).

## Description

### Technical field

The present invention relates to a vertebral stabilization system and apparatus for use in treating spinal pathologies.

The invention further relates to one or more positioning tools for use in a vertebral stabilization apparatus for treating spinal pathologies in order to position and lock the stabilization apparatus in a desired configuration for use.

### Technological background

The spinal column can be affected by various pathologies which require, in order to be resolved, a surgical intervention for stabilization.

The most common situations which may lead to such an intervention may include stenosis of the cervical tract or the lumbar tract, spondylolisthesis (also typically of the cervical tract or the lumbar tract), degenerative diseases of the intervertebral disk ("black disk") or more generally also of the vertebral bodies or the adjacent tissue (spondylosis), some forms of tumour of the spinal column, to which there may be added all cases of fractures or movements of portions of the spinal column caused by traumas.

In these cases, one of the surgical techniques most used in interventions for spinal stabilization is the percutaneous technique, so-called mini-invasive technique, which allows the insertion of the components to be fixed to the column portion involved by means of holes with relatively small dimensions.

In order to stabilize adjacent vertebrae relative to each other, it is necessary to fix in each of them a fixing screw which is fitted at the level of the respective peduncle or in a desired portion of the vertebrae to be stabilized, for example, the lamina or the vertebral body.

The fixing screws are inserted using a guide wire which is introduced into the vertebra through an access which is formed by drilling the cortical wall with a suitable tool, known as a trocar.

These stabilization apparatuses further comprise a bar or rod or other connection element which extends between the two or more fixing screws so as to maintain a defined spacing between the fixing screws and therefore between the vertebrae to which they are fixed.

The fixing screws are provided with a poly-axial head which is saddle-like and which can receive the fixing screw with the ability to tilt. The saddle-like head is further configured to receive the bar and a locking element which is intended to be screwed into the saddle-like head in order to lock the bar in the saddle-like head and to block the stabilization apparatus in a desired formation.

The locking element which is also referred to as a bolt in the jargon is usually a double-bolt type screw, that is to say, it is provided with an external thread which is intended to be engaged in a corresponding counter-thread of the saddle-like head and an internal thread which is intended to be engaged with a locking tool in order to lock the locking element in the locking position.

In order to install the vertebral stabilization apparatuses, there is initially provision for carrying out an incision in order to reach two or more vertebrae to be mutually stabilized, taking by means of a suitable tool two or more fixing screws and screwing them into the respective vertebrae. Subsequently, there is provision for inserting a bar of the desired length in the saddle-like heads of the fixing screws in such a manner that the bar extends therebetween.

The bar is cut to the desired length so as to stabilize the vertebrae in a desired position in order to carry out a stabilization, distraction or compression of the vertebrae themselves which are connected by the stabilization apparatus.

Finally, the locking element is inserted in the saddle-like head so as to lock the bar inside it and to prevent undesirable movements of the connection bar and to lock the stabilization apparatus in the desired formation.

In order to correctly carry out the stabilization, it is necessary, after inserting the fixing screw and the connection bar, to screw the locking element to the saddle-like head in order to block the mutual tilting between fixing screws and the saddle-like head and between the saddle-like head and the bar. Subsequently, there is provision for locking the locking element to the saddle-like head so as to definitively block the stabilization apparatus in the desired formation.

In order to implant the stabilization apparatuses, there are used various tools which are intended to take the portions of the apparatus and to position them in situ. Such tools comprise an active portion which is intended to engage with the portions of the apparatus to be positioned and a gripping portion which is intended to be handled by an operator.

US2019083150 sets out a vertebral stabilization system of the type described above. In this system, the locking element comprises an external thread which is provided on an internal circumference thereof and an internal thread.

There are defined on the external circumference four identical radial notches which are uniformly distributed about the external circumference of the stabilization element and which extend over a given longitudinal extent of the locking element. The radial notches define cavities for receiving an engaging tool.

There is provided in the stabilization system of US2019083150 a positioning tool for positioning the locking element comprising a hollow sleeve which is provided at a distal end thereof, that is to say, further away from an operator, with two longitudinal indentations which define on the sleeve two diametrically opposed resilient fins. The external edge of the hollow sleeve is shaped so as to define two engaging teeth which are formed so as to be inserted in the receiving cavities of the stabilization element.

In order to ensure stable engagement of the locking element by the positioning tool, the walls of the receiving cavities are provided with longitudinal channels which have a wall portion which is inclined so as to be connected in a snap-fitting manner to the engaging teeth of the positioning tool. The engaging teeth being intended to engage in a corresponding channel which is provided on the walls of the receiving cavities.

In order to take the locking element, the positioning tool is connected in the locking element until the engaging teeth engage in the channels which are provided in the receiving cavities, bringing the gripping teeth to be received in the receiving cavities. In order to lock the locking element, there is provision for appropriately rotating the positioning tool. In this manner, the engaging teeth apply a corresponding force to the lateral walls of the receiving cavities by rotating the locking element and fixing the stabilization apparatus.

The sleeve of the engaging tool has to be constructed from a material which can withstand a locking torque of at least 5 Nm in order to be able to withstand the locking torque necessary for the stabilization element.

Furthermore, the distal end of the handle which is intended to interact with the locking element has to be shaped to be connected thereto and to ensure a stable connection during the handling of the locking element.

These two characteristics make this engaging tool particularly expensive. Furthermore, the locking element also has to be shaped in order to be able to be connected to the positioning tool.

Therefore, it is necessary to define undercuts in the connection element and in the positioning tool in order to ensure a stable connection between the connection element and the positioning tool.

This complicates the construction operations of the system of US2019083150.

A disadvantage of this system is that it has high costs.

Another disadvantage is that the construction of the parts of this system, in particular the locking element and the positioning tool, are particularly difficult. US2014/214097 describes a stabilization system according to the art.

An object of the present invention is therefore to provide a stabilization system which at least partially overcomes the defects set out with reference to the cited prior art. Another object of the invention is to provide a stabilization system which has limited costs and which is simple to construct and which, at the same time, is reliable and allows stable fixing of the various parts thereof.

Another object of the invention is to provide a stabilization apparatus which at least partially overcomes the defects set out with reference to the cited prior art.

Another object of the invention is to provide a locking device which at least partially overcomes the defects set out with reference to the cited prior art.

These problems are solved by the present invention by means of a stabilization apparatus and/or a stabilization system and a tool which are constructed according to the appended claims.

In a first aspect of the invention, there is provided a vertebral stabilization apparatus comprising at least one fixing screw which is intended to be fixed to a vertebral body, a saddle-like head which is intended to receive the fixing screw in a tiltable manner and which defines a receiving seat, a locking element which is intended to be inserted in the receiving seat in order to lock the at least one fixing screw in a desired configuration, the locking element having a virtually cylindrical body which has a central cavity which extends along the longitudinal axis of the locking element, the body being provided on an external surface thereof with a positioning thread which is intended to be screwed to the receiving seat and with at least two longitudinal engagement cavities for a screwing tool, the central cavity comprising a first cavity portion which is shaped so as to define a locking seat and a second cavity portion which is positioned beside the first cavity portion in a longitudinal direction and which is delimited by a wall portion which is provided with a circumferential channel defning an engagement seat for an engaging tool of the locking element.

Advantageously, the circumferential channel is provided in a longitudinal position of the locking element which is intended to be directed during use towards a fixing screw of the stabilization apparatus.

Advantageously, the locking seat is a TORX seat or HEX seat.

Advantageously, the longitudinal engagement cavities extend over a portion of the longitudinal extent of the locking element which has such dimensions that at least one additional longitudinal portion of the locking element is void of the longitudinal cavities. The positioning thread is provided in the region of the additional longitudinal portion. Advantageously, the additional longitudinal portion has such an extent that the positioning thread carries out at least one complete revolution.

In a second aspect of the invention, there is provided an engaging tool for a locking element of a vertebral stabilization apparatus comprising an actuation portion which is intended to be manoeuvred by an operator and a rod which is provided at a distal end thereof which is opposite the actuation portion with a plurality of engaging flaps which extend in a longitudinal direction and which are provided at a free end thereof with a respective projection which projects in a radial direction from each engaging flap and which acts as an engaging element for the locking element, the projection being formed so as to be connected in a positive-locking manner to an engagement cavity of the locking element.

Advantageously, the engaging tool is configured to be used with the stabilization apparatus of the invention.

In a preferred version, the plurality of engaging flaps preferably comprise two engaging flaps which are mutually opposite in a radial direction.

The engaging flaps of the plurality of engaging flaps are movable between a rest configuration, in which the engaging flaps are mutually spaced apart by a first distance, an engaging configuration, in which the flaps are mutually spaced apart by a second distance less than the first distance and the projections are received in the receiving cavity, and an insertion configuration, in which the engaging flaps are spaced apart by a third distance less than the first distance and the second distance in order to be able to insert the engaging tool in the locking element.

In another aspect of the invention, there is provided a vertebral stabilization system comprising a stabilization apparatus according to the invention and a plurality of fixing tools for fixing the stabilization apparatus, wherein the plurality of fixing tools comprise an engaging tool according to the invention.

In the present description and the appended claims, a portion of an element is said to be "proximal" or "distal" when during use this portion is nearer or further away from the operator, respectively.

The term "end region" of an element is intended to be understood to be an element portion which comprises the end and the region near this end, preferably up to a distance between 1 and 5 mm from this end.

### Brief description of the drawings

The features and advantages of the invention will be better appreciated from the detailed description of a preferred embodiment thereof which is illustrated by way of non-limiting example with reference to the appended drawings, in which:
- Figure 1 is a schematic view of a portion of a vertebral stabilization system according to the invention;
- Figures 2 and 3 are two schematic views of a portion of a vertebral stabilization apparatus according to the invention with and without a connection bar, respectively;
- Figure 4 is a perspective view of a locking element of the invention;
- Figure 5 is a sectioned view of the locking element of Figure 4;
- Figures 6 and 7 are a perspective view and a front cross-sectional view of a screwing tool and an engaging tool according to the invention;
- Figure 7A is a broken-away enlarged view of a portion of Figure 7;
- Figure 7B is an enlarged view of the detail B of Figure 7A;
- Figure 8 is a broken-away, exploded view of the engaging tool of Figure 6 in a different operating configuration and of a locking element of the invention;
- Figure 9 is a front view of the screwing tool according to the invention which is connected to a locking element of the invention;
- Figure 9A is a broken-away, enlarged cross-sectional view of a detail of the screwing tool of Figure 9;
- Figure 9B is an enlarged view of a detail of the screwing tool of Figure 9, which shows the connection to a locking element of the invention;
- Figure 9C is an enlarged view of a detail of the screwing tool of Figure 9 without the locking element;
- Figure 10 is an exploded front view of a locking tool of the invention and the locking element of the invention;
- Figure 10A is an enlarged cross-section of a detail of the locking tool of Figure 10;
- Figure 10B is an enlarged view of a detail of the locking tool of Figure 10 which shows the connection to a locking element of the invention.

### Preferred embodiment of the invention

In the appended Figures, 100 generally designates a spinal stabilization system which is constructed according to the present invention.

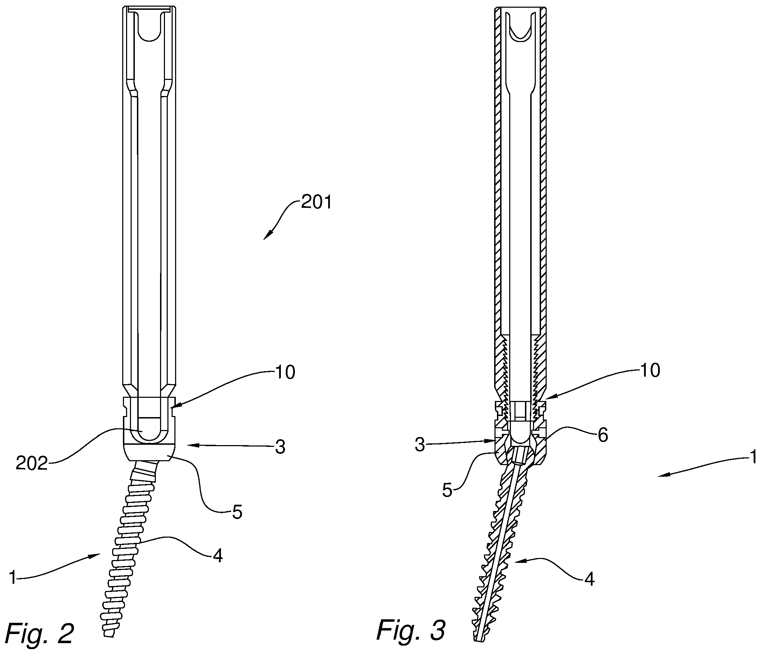

The stabilization system 100 comprises a stabilization apparatus 200 which is intended to be fixed to one or more vertebrae to be stabilized and which is shown in greater detail in Figures 2 and 3 and a plurality of tools which are intended to interact with various portions of the stabilization apparatus 200 in order to insert them and to fix them, as better explained below.

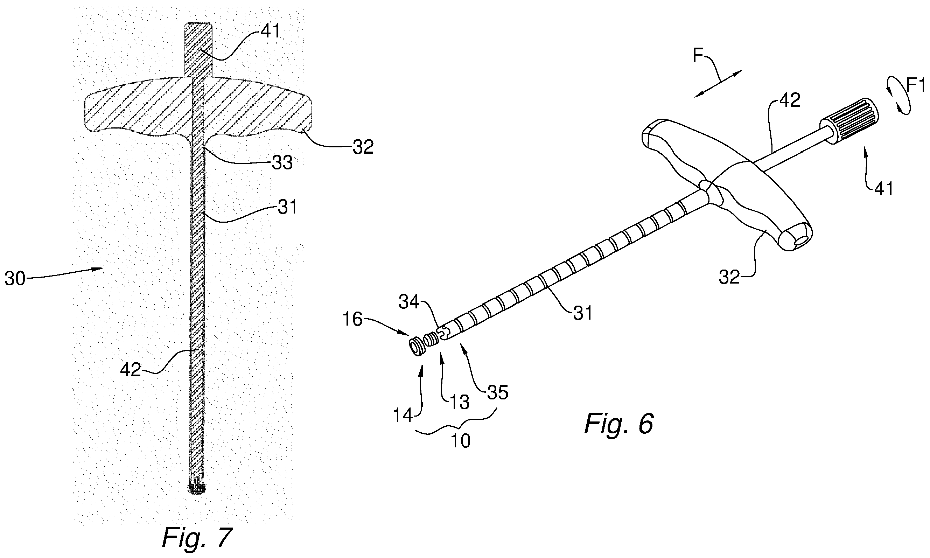

The plurality of tools comprises a screwing tool 30, which is shown in Figures 6, 7, 9, 9A-C and which has a handle 32 and a hollow sleeve 31 which is fixed to the handle 32. The handle 32 is provided with a hole 33 which communicates with the hollow sleeve 31 in order to define an insertion cavity 31A which extends along the longitudinal axis X of the screwing tool 30. The insertion cavity 31A is formed so as to be able to insert/disengage, by being moved in both directions of the arrow F, various tools into/out of the insertion cavity 31A of the screwing tool 30 in order to install the stabilization apparatus 200, as better explained below.

The stabilization apparatus 200 in a general form thereof comprises at least a first fixing device 201 and a second fixing device, which is not shown in the Figures, the first fixing device 201 and a second fixing device being intended to be fixed in two different vertebral bodies to be stabilized, and a connection element 202 which extends between the first fixing device 201 and the second fixing device.

Although reference is made below only to a stabilization apparatus comprising two fixing devices, in other versions of the stabilization apparatus which are not shown there may be provided stabilization apparatuses which have a number of fixing devices different from two in accordance with the number of vertebrae to be stabilized with the stabilization apparatus of the invention.

The fixing devices of the stabilization apparatus 200 are virtually mutually identical, so that only one will be described and shown for the sake of brevity.

The fixing device 201 comprises a fixing screw 1 which is intended to be fixed to a vertebral body, a saddle-like head 3 and a locking element 10 for locking and stabilizing the stabilization apparatus 200 in a desired position, as better explained below.

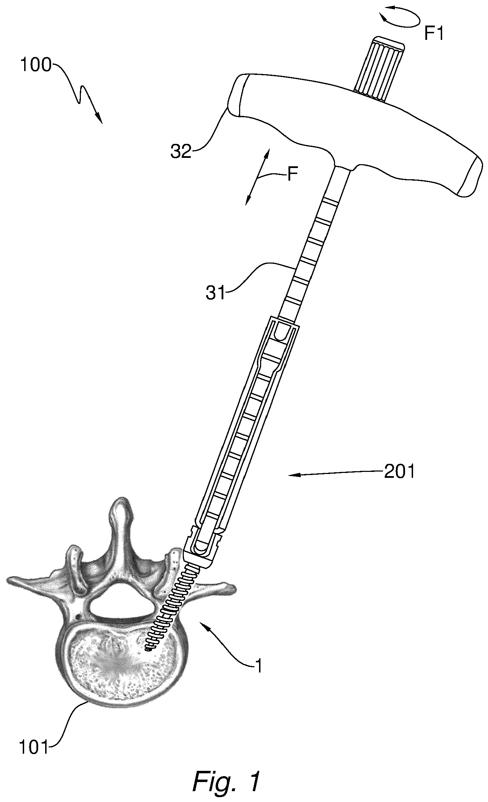

The fixing screw 1 is intended to be fixed to a vertebral body 101, as schematically shown in Figure 1, in a desired zone thereof, preferably the peduncle or the lamina, in accordance with the access which is used for inserting the stabilization apparatus of the invention and/or the pathology to be treated with the stabilization apparatus of the invention.

In some cases, there may be provision for inserting two different stabilization apparatuses in the same vertebra by fixing the fixing screws to the opposite sides of the vertebrae to be fixed.

In the version shown in the Figures, the connection element is a connection bar 202 which can be seen in Figure 2 and which has a circular cross-section, but in other versions of the stabilization apparatus of the invention there may be provided a rod or other connection element which extends between the two or more fixing screws.

The connection element 202 is intended to maintain a defined distance between the fixing screws 1 of the stabilization apparatus 200 and, therefore, between the vertebrae to which they are fixed.

The connection element is cut to the desired length depending on the effect which it is desirable to obtain with the stabilization apparatus of the invention: stabilization, distraction or compression.

The fixing screw 1 comprises a threaded rod 4 which is intended to be screwed into the vertebral body 101 and a head 5 which has a thread 6 which defines a seat for a screwing device in order to screw the fixing screw 1 to the vertebral body.

The thread 6 is of the type known in the field.

The rod 4 of the fixing screw is selected with a length suitable for the objective and/or the vertebral body to which it has to be screwed.

The fixing device 201 further comprises a saddle-like head 3 which can be positioned so as to receive the head 5 of the fixing screw 1 with the ability to tilt.

The saddle-like head 3 is formed so as to define a receiving seat 7 in which there are received, as better explained below, the head 5 of the fixing screw 1, the locking element 10 and the connection element 202. The saddle-like head 3 is provided with a through-hole 8, in which the threaded rod 4 of the fixing screw 1 is inserted until the head 5 is brought into abutment against the walls of the through-hole 8.

The through-hole 8 is formed in such a manner that the fixing screw 1 can be tilted inside it in order to be able to orientate the stabilization apparatus 200.

The receiving seat 7 is U-shaped and is delimited by threaded walls 7A, on which the locking element 10 is screwed in order to lock the stabilization apparatus 200, as better explained below.

The locking element 10 is inserted in the receiving seat 7 in such a manner that the connection element 202 is interposed between the locking element 10 and the head 5 of the fixing screw 1, as can be seen in Figure 2.

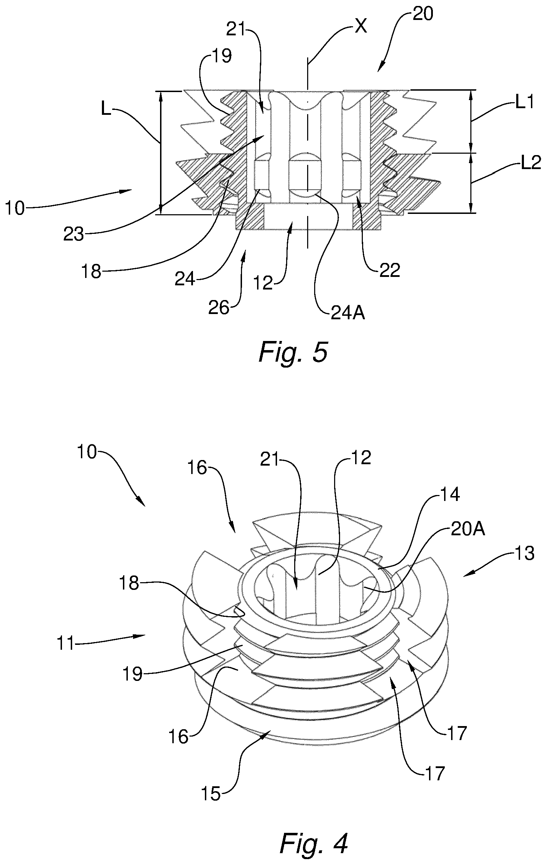

The locking element 10, which is also referred to in the jargon as a bolt and which is shown in greater detail in Figures 4 and 5, comprises a virtually cylindrical body 11 with a central cavity 12 which extends along the longitudinal axis X of the locking element 10.

The member 11 comprises an external member 13 and an internal member 14 which is received inside the external member 13 with the ability to rotate, the internal member 14 and the external member 13 being rotatable with respect to each other about the longitudinal axis X of the member 11.

The external member 13 is of annular form with a length "L" in the longitudinal direction and is provided on an external wall 13A thereof with a positioning thread 15 which is intended to be screwed on the threaded wall 7A of the receiving seat 7 in order to screw the locking element 10 in the receiving seat 7.

Longitudinal indentations 17 are provided on the external wall 13A of the external member 13, the longitudinal indentations 17 defining four longitudinal engagement cavities 16 for the screwing tool 30, as better explained below. The longitudinal engagement cavities 16 are spaced apart over the circumference of the external wall 13A.

In other versions of the locking element which are not shown, there is defined on the external wall a number of longitudinal cavities other than four, for example, two or three.

Preferably, the longitudinal engagement cavities 16 are uniformly spaced about the circumference of the external wall 13A.

This allows the engagement and the actuation of the screwing tool 30 to be promoted.

The longitudinal engagement cavities 16 have a longitudinal extent L1 less than the length "L" of the external member 13.

In this manner, for an additional longitudinal extent L2 of the external member 13, there remains defined on the external wall 13A a portion of the positioning thread 15 which is not interrupted by the longitudinal engagement cavities 16 and which extends over the entire circumference of the external wall 13A.

In other words, in the length "L" of the external member 13 there is defined a longitudinal extent L1 which is provided with the longitudinal engagement cavities 16 and an additional longitudinal extent L2 without the longitudinal engagement cavities 16.

This allows the positioning of the locking element 10 in the receiving cavity 7 to be made more stable.

Advantageously, the additional longitudinal extent L2 is such that the positioning thread 15 makes at least one complete revolution in the additional longitudinal extent L2.

The internal wall 13B of the external member 13 is provided with a connection thread 18 which is intended to be connected to an additional connection thread 19 which is provided on an external face 14A of the internal member 14 in order to mutually connect the external member 13 and internal member 14 and to be able to screw/unscrew the external member 13 and internal member 14 with respect to each other.

The internal member 13 also has an annular form, on the internal wall 20 of which the additional connection thread 19 is provided and the internal face 13B of which delimits and defines the central cavity 12 of the locking element 10.

The external member 13 is further provided with a base 26 which is intended to be directed during use towards the connection element 202 and to abut against the connection element 202, as better explained below.

The internal member 14 can be screwed in the external member 13 in order to move the base 26 in a longitudinal direction X in order to lock the connection element 202, as better described below.

The internal wall 20 of the central cavity 12 comprises a first wall portion and a second wall portion 20A, 20B which are subsequent along the longitudinal axis X and which define a first cavity portion 21 and a second cavity portion 22 which are beside each other along the longitudinal axis X, respectively.

The first wall portion 20A is shaped so that the first cavity portion 21 defines a TORX seat 23. In other versions, the first cavity portion 21 defines a locking seat 23 of the HEX type.

The first wall portion 20A is, in other versions which are not shown, shaped so as to define a locking seat which is formed so as to receive a tool which applies a high screwing torque, limiting or preventing the undesirable disengagement of the tool. There is provided in the second wall portion 20B a circumferential channel 24A which extends along the circumference of the second wall portion 20B and which defines an engagement cavity 24 for an engaging tool 40.

The channel 24A is recessed with respect to the second wall portion 20B and is C-shaped and defines an engagement seat for an engaging tool 40 for the locking element 10, as better explained below.

The internal wall 20 is formed in such a manner that there is defined between the channel 24A and the locking seat 23 an abutment 25 which can block the sliding of the engaging tool 40 in a longitudinal direction X out of the engagement cavity 24 towards the first cavity portion 21, as better explained below.

In the version shown, the engagement cavity 24 is defined by a single circumferential channel 24A which extends over the entire circumference of the second wall portion 20B.

In other versions which are not shown, there may be provided in the locking element two or more channel portions which each extend over a predetermined circumference section of the second wall portion and which define different engagement cavity portions.

The engagement cavity portions are preferably uniformly spaced apart over the circumference of the second wall portion and/or have an almost identical extent. Preferably, the cavity portions are provided in mutually opposite positions.

This allows the engagement by the engaging tool 40 of the locking element 10 to be made more stable.

The engagement cavity 24 has a through-hole which is greater than the hole of the locking seat 23.

With reference to Figures 6 to 10, there are shown a number of tools of the plurality of tools to be used to implant the stabilization apparatus 200 of the invention.

This plurality of tools comprises the screwing tool 30, in the insertion cavity 31A of which there can be inserted various tools which are intended to interact with the various portions of the stabilization apparatus 200 and the handle 32 of which can be used to handle the tools of the plurality of tools.

As set out above, the insertion cavity 31A is formed in such a manner that the tools can be moved inside the insertion cavity 31A in a longitudinal direction in both directions of the arrow F and/or rotated as indicated by the arrow F1.

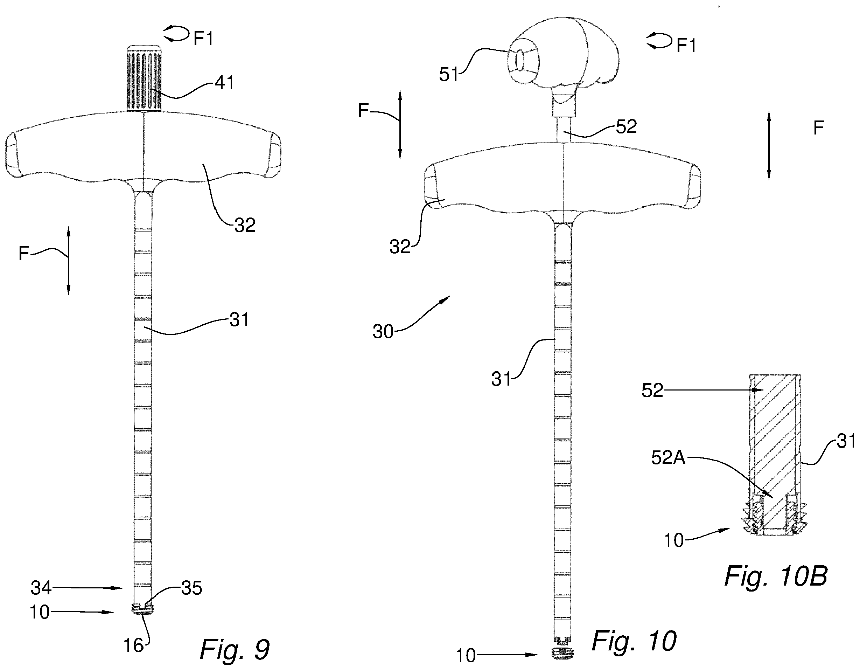

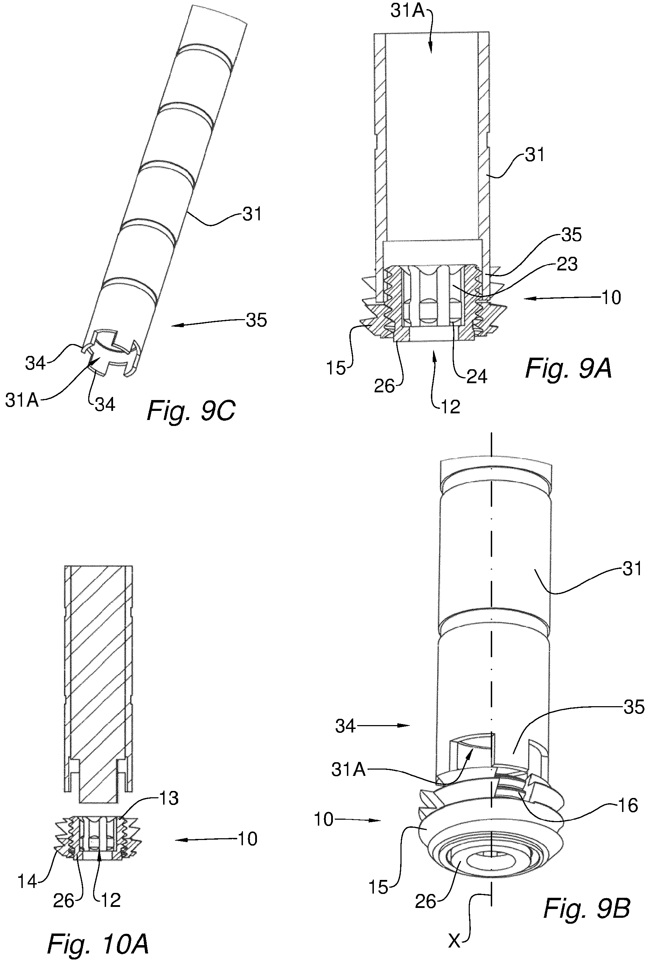

As can better be seen in Figure 9C, the distal end 35 of the hollow sleeve 31 is provided with engaging teeth 34 which extend in a longitudinal direction from the distal end 35 of the hollow sleeve 31 and which are intended to be inserted in the corresponding longitudinal engagement cavities 16 of the locking element 10.

The number and/or form of the engaging teeth 35 is selected in such a manner that the engaging teeth 35 are paired with the longitudinal engagement cavities 16, as clearly shown in Figures 9A and 9B.

In this manner, it is possible with the screwing tool 30 to screw the locking element 10 to the receiving seat 7, as explained below.

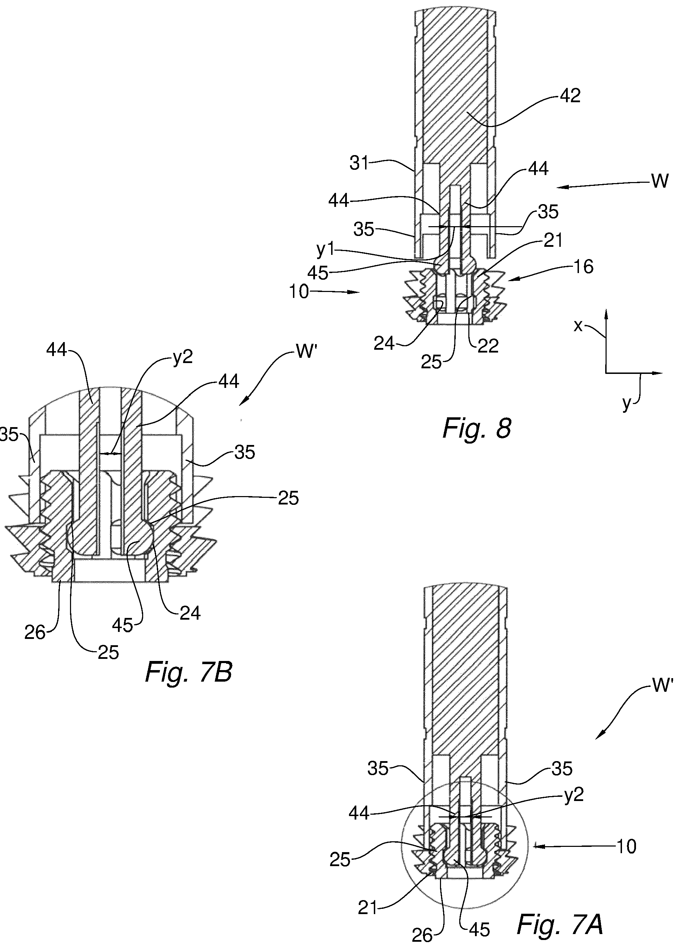

The plurality of tools further comprises an engaging tool 40 which is shown in greater detail in Figures 6, 7, 7A-7B and 8, comprising an actuation portion 41 and a rod 42 which is fixed to the actuation portion 41 and which has a virtually cylindrical formation which is formed so as to be received with an ability to slide inside the longitudinal cavity 31A of the hollow sleeve 31 until the actuation portion 41 is brought into abutment against the handle 32 of the screwing tool 30, as shown in Figure 7.

The rod 42 is slidable in the longitudinal direction of the hollow sleeve 31 in both directions of the arrow F.

The rod 42 is provided at a distal end 42A thereof opposite the actuation portion 41 with an engaging device 43 which is intended to be connected to the engagement cavity 24 of the locking element 10 in order to take and position the locking element 10, as better explained below.

The engaging device 43, which can better be seen in Figures 7A, 7B and 8, projects from the rod 42 in a longitudinal direction X and comprises two engaging flaps 44 which are provided in an opposite position and which are provided at a free end thereof with a respective projection 45 which projects in the radial direction Y from the engaging flaps 44 and which acts as an engaging element for the locking element 10.

The projections 45 are formed so as to be connected in a positive-locking manner to the engagement cavity 24 of the locking element 10, as better explained below.

The engaging flaps 44 are spaced apart from each other by a distance Y1 and are configured so as to be able to be flexed in a radial direction in order to be mutually moved together and to be inserted inside the locking element 10. The distance Y1 is usually between 0.25 mm and 0.5 mm.

The engaging device 43 is constructed from a material having a specific resilience in order to allow the tiltability of the engaging flaps 44. Preferably, the engaging device 43 is constructed from superelastic Nitinol.

The engaging device 43 of the engaging tool 40 is movable at least between a rest configuration W, in which the engaging flaps 44 are spaced apart by the distance Y1, an engaging configuration W', in which the engaging flaps 44 are spaced apart by the second distance Y2 less than the first distance Y1 and the projections 45 can be received in the receiving cavities 24, and an insertion configuration which is not shown and in which the engaging flaps 44 are spaced apart by a third distance Y3 less than the second distance Y2 and in which the projections 45 are positioned in the region of the first cavity portion 21, that is to say, of the locking seat 23.

In a version, the engaging flaps 44 are mutually in contact in the insertion configuration.

The engaging device 43 is further formed so that, in the rest configuration W, the spatial requirement in cross-section of the projections 45 is greater than the through-hole of the first cavity portion 21 and the second cavity portion 22. Therefore, during the insertion of the engaging tool 40 into the locking element 10, the engaging flaps 44 are compressed in a radial direction and moved towards each other, as explained below.

The engaging device 43 is further formed so that, in the engaging configuration W', the spatial requirement in cross-section of the projections 45 is greater than the through-hole of the first cavity portion 21.

The engaging device 43 is formed so that, in the rest position, the spatial requirement in cross-section of the projections 45 is greater than the through-hole of the first cavity portion 21 and, preferably, than the second cavity portion 22.

In the engaging configuration, therefore, the engaging flaps 44 urge the projections 45 into the engagement cavity 24.

This allows the engagement of the locking element 10 by means of the engaging tool 40 to be stabilized, as better explained below, and undesirable unscrewing actions of the engaging tool 40 to be prevented.

The presence of the abutment 25 further allows undesirable disengagement of the projections from the engagement cavity 24 to be prevented.

When the engaging tool 40 is inserted into the central cavity 12, the engaging flaps 44 are compressed in the direction towards each other by inserting the engaging tool 40 until the projections 45 are brought into the region of the engagement cavity 24, the projections 45 are received in the engagement cavity 24 in a state urged by the engaging flaps 44 which widen in order to seek to return to the rest position. This allows a stable connection of the locking element 10 to the engaging tool 40.

The projections 45 are formed so as to be connected in a positive-locking manner to the engagement cavity 24.

Advantageously, the projections 45 and the engagement cavity 24 have such dimensions, respectively, that, in the engaging configuration W', when the projections 45 are received in the engagement cavity 24, the flaps 44 are spaced apart by an additional distance Y2 of from 65% to 95% of the distance Y1 taken up in the rest configuration W, that is to say, when the engaging tool 40 is not inserted in the stabilization element 10.

The resilient return thrust of the engaging flaps 44 allows stable connection of the projections 45 to the engagement cavity 24.

In other versions which are not shown, the engaging tool 40 is provided with a number of engaging flaps different from two, for example, 3 or 4. Preferably, the engaging flaps are provided in a mutually opposite position so as to make the engagement of the locking element 10 by the engaging tool 40 more stable. Each engaging flap 44 being provided with a respective projection 45.

Since the radial spatial requirement of the projections 45 in the rest configuration W is greater than the aperture of the first cavity portion 21, by inserting the engaging tool 40 in the first cavity portion 21 the engaging flaps 44 are mutually moved together in order to reduce the overall spatial requirement.

As a result of the flexibility of the engaging flaps 44 and the fact that, in a rest condition, the engaging flaps 44 are mutually spaced apart, the engaging tool 40 can be inserted in the first cavity portion 21.

When the engaging tool 40 is progressively inserted in the central cavity 12, the projections 45 are progressively moved towards the engagement cavity 24. When the projections 45 move into the region of the engagement cavity 24, and the engaging tool 40 is in the engaging configuration W' under the action of the resilient thrust, the projections 45 are urged against the engagement cavity 24 in such a manner that the projections 45 are received in the engagement cavity 24.

Therefore, there is produced a stable engagement of the locking element 10 with the engaging tool 40.

The plurality of tools further comprises a locking tool 50 which is shown in greater detail in Figures 10 and 10A and which is intended to be connected to the locking seat 23 of the locking element 10 in order to lock the locking element 10 in the receiving cavity 7, as better explained below.

The locking tool 50 comprises an actuation handle 51 and an additional rod 52 which projects from the actuation handle 51 and which has a virtually cylindrical formation which is formed so as to be received with the ability to slide inside the insertion cavity 31A of the hollow sleeve 31. The additional rod 52 can slide in both directions of the arrow F in order to be inserted until the actuation handle 51 is brought into abutment against the handle 32 of the screwing tool 30 and it is further rotatable in the insertion cavity 31A in order to screw the locking element 10.

The additional rod 52 is provided at a distal end 52A thereof, which is opposite the actuation handle 51, with a locking device 53 which is intended to be connected to the locking seat 23 of the locking element 10 in order to lock the locking element 10 in the receiving cavity 7, as better explained below.

The locking device 53 is configured so as to be connected in a positive-locking manner to the locking seat 23. In this manner, by acting on the actuation handle 51, it is possible to screw the locking element 10 in the receiving seat 7.

Advantageously, the locking device 53 is provided with a locking head which is intended to be connected in a positive-locking manner to the locking seat 23. Advantageously, the locking head is of the TORX or HEX type.

During operation, after generating the access to a vertebral body 101, there is initially provision for fixing the fixing screw 1 in the vertebral body 101 by means of a suitable fixing tool, subsequently the connection element 202 is inserted in the receiving seat 7 of the fixing screw 1.

Subsequently, the locking element 10 is inserted in the receiving cavity 7 by means of the engaging tool 40.

To do so, the engaging tool 40 is inserted in the central cavity 12, by connecting the projections 45 to the engagement cavity 24. With the engaging tool 40 in an engaging configuration W', there is provision for inserting the locking element 10. Subsequently, the engaging tool 40 is extracted from the sleeve 31 by extracting the engaging device 43 from the locking element 10. Since the external member 13 and the internal member 14 are connected to each other, and the engaging flaps 44 are resiliently deformable, a limited force is sufficient to extract the engaging device from the locking element 10.

Subsequently, there is provision for screwing the external member 13 of the locking element 10 to the threaded walls 7A of the receiving cavity 7, the sleeve 31 is connected to the external member 13 by inserting the engaging teeth 35 into the longitudinal cavities 16 until they are brought into abutment against the base wall 16A of the longitudinal engagement cavities 16.

By acting on the screwing tool 30, the external member 13 is screwed to the threaded walls 7A of the receiving seat 7, thereby blocking the tilting of the fixing screw 1 with respect to the receiving seat 7.

Therefore, the position of the stabilization apparatus 200 is stabilized.

After stabilizing the stabilization apparatus 200, there is provision for locking it.

To this end, the locking tool 50 is inserted into the screwing tool 30 until the locking device 53 is brought into the region of the locking seat 23 of the screwing tool 30.

After connecting the locking device 53 to the locking seat 23, by carrying out a rotation in the direction of the arrow F1 the internal member 14 is screwed onto the external member 13 so as to urge the internal member 14 towards the connection element 202.

By rotating the internal member 14, the base 26 is progressively brought towards the connection element 202 and into abutment against the connection element 202.

By continuing to screw the internal member 14, the connection element 202 is locked inside the receiving seat 7 between the locking element 10 and the head 5 of the fixing screw 1.

Therefore, the present invention solves the problem set out above with reference to the cited prior art, by increasing the degree of reliability correlated with the interventions of spinal stabilization.

Naturally, the person skilled in the art may provide for a number of modifications and variants, both structural and functional, to the embodiment described above by way of example. For example, the guide wire may be constructed from different materials and may have different forms and dimensions or, furthermore, the device may be used in surgical interventions other than spinal stabilization.

## Claims

1. Vertebral stabilization apparatus (200) comprising at least one fixing screw (1) which is intended to be fixed to a vertebral body (101), a saddle-like head (3) which is intended to receive said fixing screw (1) in a tiltable manner and which defines a receiving seat (7), and a locking element (10) which is intended to be inserted in said receiving seat (7) in order to lock said at least one fixing screw (1) in a desired configuration, said locking element (10) having a virtually cylindrical body (11) which has a central cavity (12) extending along the longitudinal axis (X) of the locking element (10), said body (11) being provided on an external surface thereof with a positioning thread (15) which is intended to be screwed to said receiving seat (7) and with at least two longitudinal engagement cavities (16) for a screwing tool (30), **characterized in that** the central cavity (12) comprises a first cavity portion (21) which is shaped so as to define a locking seat (23) and a second cavity portion (22) which is positioned beside said first cavity portion (21) in a longitudinal direction (X) and which is delimited by a wall portion (20B) which is provided with a circumferential channel (24) defining an engagement seat (24) for an engaging tool (40, 45) of said locking element (10).

2. Stabilization apparatus according to the preceding claim, wherein said second cavity portion (22) comprises two different circumferential channels which are provided in mutually opposite positions and which define different engagement seats (24) for an engaging tool (40, 45) of the locking element (10), said different circumferential channels each preferably extending over a corresponding circumferential portion at an angle between 90 and 180°.

3. Stabilization apparatus according to claim 1, wherein said second cavity portion (22) comprises a single circumferential channel which extends over the entire circumference of the wall of said second cavity portion (22).

4. Stabilization apparatus according to any one of the preceding claims, wherein said locking seat (23) is of the TORX or HEX type.

5. Stabilization apparatus according to any one of the preceding claims, wherein the locking element (10) comprises an external member (13) which is provided with the positioning thread (15) and an internal member (14) which is connected to the external member (13) and which can be screwed with respect to the external member (13) and which defines the central cavity (12) of said locking element (10).

6. Stabilization apparatus according to any one of the preceding claims and further comprising a connection element (202) which is inserted in said receiving head (7) so as to be interposed between a head (5) of said fixing screw (1) and said locking element (10).

7. Stabilization apparatus according to any one of the preceding claims and further comprising at least one second fixing screw which is intended to be fixed to an additional vertebral body, the additional fixing screw being provided with a corresponding receiving head in which said connection element is configured to extend between said receiving head (7) and said additional receiving head in order to connect said fixing screw (1) and said additional fixing screw in order to maintain said vertebral body and said additional vertebral body with desired mutual spacing.

8. Engaging tool (40) for a locking element (10) of a vertebral stabilization apparatus (200) according to any one of claims 1 to 7 and comprising an actuation portion (41) which is intended to be manoeuvred by an operator and a rod (42) which is provided at a distal end (42A) thereof which is opposite the actuation portion (41) with a plurality of engaging flaps (44) which extend in a longitudinal direction (X) and which are provided at a free end thereof with a respective projection (45) which projects in a radial direction (Y) from each engaging flap (44) and which acts as an engaging element for a locking element (10), said projection (45) being formed so as to be connected in a positive-locking manner to an engagement cavity (24) of said locking element (10).

9. Engaging tool according to claim 8, wherein said plurality of engaging flaps comprises two engaging flaps (44) which are mutually opposite in a radial direction (Y).

10. Engaging tool according to claim 8 or 9, wherein the engaging flaps (44) of said plurality of engaging flaps (44) are mutually spaced apart by a distance (Y1) so as to be able to be flexed in a radial direction in order to be inserted inside the locking element (10).

11. Engaging tool according to any one of claims 8 to 10, wherein the engaging flaps (44) of said plurality of engaging flaps (44) form an engaging device (43) of the engaging tool (40), the engaging device (43) being constructed from a material having a specific resilience in order to allow the tiltability of the engaging flaps (44), wherein said engaging device (43) is preferably constructed from superelastic Nitinol.

12. Vertebral stabilization system (100) comprising a stabilization apparatus (200) according to any one of claims 1 to 7 and a plurality of fixing tools for fixing said stabilization apparatus (200), said plurality of fixing tools comprising an engaging tool according to any one of claims 8 to 11.
